# EUROPEAN PATENT APPLICATION

(11) **EP 2 228 089 A1**
(43) Date of publication of application: **15.09.2010**
(21) Application number: 09154668.9
(22) Date of filing: 09.03.2009
(51) Int. Cl.: A61M 21/00, A61M 21/02, B64D 11/00

(54) **Method and system for alleviating motion sickness with a passenger of a moving vehicle**

(71) Applicant: Nederlandse Organisatie voor Toegepast -Natuurwetenschappelijk Onderzoek TNO, 2628 VK Delft (NL)
(72) Inventor: van Gent, Ronald Nikola Huib Willem, 3768 GN Soest (NL); Bos, Jelte Egbert, 3972 GT Driebergen (NL)
(74) Representative: Hatzmann, Martin

(57) **Abstract**

Method and system for alleviating motion sickness with a passenger of a moving vehicle, comprising means (1) for detecting the vehicle's motions, means (2, 3) for detecting conditions which may influence the vehicle's future motions, means (4) for estimating, based on the detected vehicle motions, the detected conditions, and/or the vehicle's own characteristics, the vehicle's estimated future motions and means (6) for visually displaying to the passenger, in a form adapted to the passenger's perception of his/her location, a visual image representing the vehicle's present and estimated future spatial conditions. The means for visually displaying to the passenger a visual image may represent the vehicle's present and estimated future spatial conditions, comprising a visual display mainly in front of the passenger. The visual image representing the vehicle's present and estimated future spatial conditions may be represented by a (pseudo)3D image of a virtual path, channel or tunnel, having a course which represents the course of the vehicle's estimated future motions.

## Description

The invention concerns a method and system for alleviating motion sickness with a passenger of a moving vehicle, e.g. an airplane, a car, a ship etc.

Motion sickness or kinetosis is a condition in which a disagreement exists between visually perceived self motion and attitude with respect to gravity, the vestibular system's sense thereof and/or what the central nervous system expects based on previous experiences. Depending on the environment it can also be referred to as seasickness, carsickness, simulation sickness, airsickness, or space sickness.

About one third of people are susceptible to motion sickness even in mild circumstances such as being on a boat in calm water, although more than two third of people are susceptible in more severe conditions.

The present invention is based on the understanding that not only information about the current motion is important regarding motion sickness, but also is the motion that will be made in the near future. This is exemplified by the fact that passengers looking forward generally suffer less than passengers looking backwards to the motion, as well as the fact that the driver, being able to control and thus anticipate even better on the motions to come, generally does not suffer at all, while the passive passenger typically suffers most. Lack of information of the passenger about the motions that the vehicle is to be expected to make (i.e. future motions) is thus an important contributor to motion sickness.

It is one aim of the invention to provide a method for alleviating or counteracting motion sickness with a passenger of a moving vehicle.

It is another aim of this invention to provide a system for alleviating motion sickness with a passenger of a moving vehicle.

It is yet another aim of the invention to provide a system for alleviating motion sickness with a passenger of a moving vehicle which is or can be integrated with system components already present in the relevant vehicle.

The method for alleviating motion sickness with a passenger of a moving vehicle according to the present invention, preferably comprise following steps:
- detecting the vehicle's motions;
- detecting conditions which may influence the vehicle's future motions;
- estimating (or predicting), based on the detected vehicle motions and/or the detected conditions, the vehicle's future motions;
- visually displaying to the passenger, in a form adapted to the passenger's perception of his/her location, a visual image representing the vehicle's present and estimated (predicted) future spatial conditions.

By visually displaying to the passenger, in a form adapted to the passenger's perception of his/her location, a visual image representing the vehicle's present and estimated future spatial conditions will meet the lack of information of the passenger about the future motions, causing alleviation of motion sickness analogously to a forward looking passenger in a car as opposed to the one lacking this anticipatory information.

The conditions which may influence the vehicle's future motions may be detected from any vehicle steering system and/or a vehicle navigation or sensing system.

In a preferred embodiment the visual image representing the vehicle's present and estimated future spatial conditions may be represented by a 3D image of a virtual path, channel or tunnel, having a course which represents the course of the vehicle's estimated future motions.

A system for alleviating motion sickness with a passenger of a moving vehicle, preferably comprises means for detecting the vehicle's motions, means for detecting conditions which may influence the vehicle's future motions, means for estimating (predicting), based on the detected vehicle motions and/or the detected conditions, the vehicle's estimated future motions and means for visually displaying to the passenger, in a form adapted to the passenger's perception of his/her location, a visual image representing the vehicle's present and estimated future spatial conditions.

Preferrably, the means for visually displaying to the passenger a visual image representing the vehicle's present and estimated future spatial conditions, comprises a visual display mainly in front of the passenger. Such a visual display can be integrated with system components already present in the relevant vehicle, in particular visual displays which already are in use for displaying movies, DVDs etc. in modern airplanes, cars etc. Peripheral displays, however, may be used as well and are assumed to contribute positively to the effectiveness of the method.

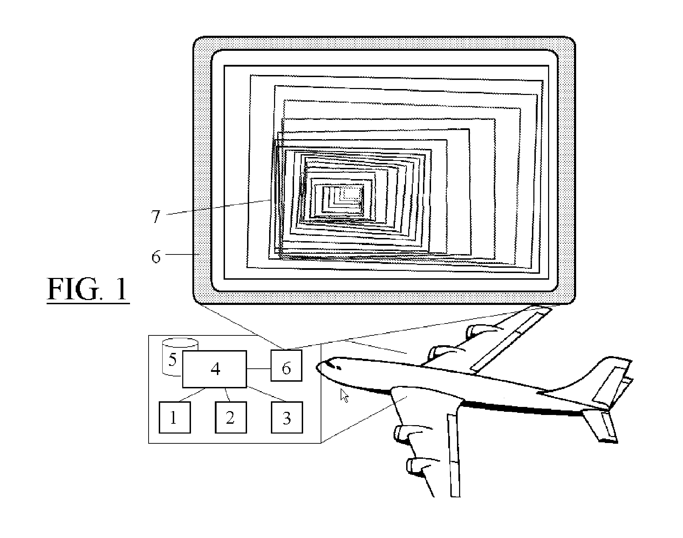

Hereinafter the invention will be elucidated with reference to figure 1, which shows an exemplary embodiment of a system according to the invention.

The exemplary system for alleviating motion sickness with a passenger of a moving vehicle, as shown in figure 1, comprises means for detecting the vehicle's motions, constituted by motion or acceleration detectors, represented by a module 1, means for detecting conditions which may influence the vehicle's future motions, constituted by a module 2 arranged for detecting the future path, e.g. derived from the vehicle's steering system and/or data from a satellite based vehicle navigation system, and/or a module 3 arranged for detecting external influences like wind (relevant for ships and airplanes) and/or water waves and/or streams (relevant for ships). Means for estimating, based on said detected vehicle motions (module 1) and/or said detected conditions (modules 2 and 3), the vehicle's estimated future motions are constituted by a computer of processor 4. In the estimation of the vehicle's estimated future motions the vehicle's specific behaviour and characteristics e.g. the vehicle's responses to the driver's (car), pilot's (airplane) or helmsman (ship) steering actions and/or external forces (wind, stream etc.) due to the vehicle's mass, size and specific dimensions, are taken into account by storing and retrieving the relevant characteristic behaviour parameters in a database 5.

Means for visually displaying to the passenger, in a form adapted to the passenger's perception of his/her location, a visual image representing the vehicle's present and estimated future spatial conditions are constituted by a display screen 6 which may be mounted (e.g. in the backside of a seat) in front of the passenger. The visual image representing the vehicle's present and estimated future spatial conditions (i.e. particularly the vehicle's future path and motions) can e.g. be represented -as shown in figure 1- in the form of a (pseudo)3D tunnel like pattern of rectangles.

## Claims

1. Method for alleviating motion sickness with a passenger of a moving vehicle, comprising following steps:
- detecting the vehicle's motions;
- detecting conditions which may influence the vehicle's future motions;
- estimating or predicting, based on said detected vehicle motions and/or said detected conditions, the vehicle's future motions;
- visually displaying to the passenger, in a form adapted to the passenger's perception of his/her location, a visual image representing the vehicle's present and estimated future spatial conditions.

2. Method according to claim 1, wherein said conditions which may influence the vehicle's future motions are detected from any vehicle steering system.

3. Method according to claim 1, wherein said conditions which may influence the vehicle's future motions are detected from any vehicle navigation or sensing system.

4. Method according to claim 1, wherein said visual image representing the vehicle's present and estimated future spatial conditions is represented by a 3D or pseudo 3D image of a virtual path, channel or tunnel, having a course which represents the course of the vehicle's estimated future motions.

5. System for alleviating motion sickness with a passenger of a moving vehicle, comprising means (1) for detecting the vehicle's motions, means (2, 3) for detecting conditions which may influence the vehicle's future motions, means (4) for estimating, based on said detected vehicle motions and/or said detected conditions, the vehicle's estimated future motions and means (6) for visually displaying to the passenger, in a form adapted to the passenger's perception of his/her location, a visual image representing the vehicle's present and estimated future spatial conditions.

6. System according to claim 5, said means for visually displaying to the passenger a visual image representing the vehicle's present and estimated future spatial conditions, comprising a visual display mainly in front of the passenger, but possibly also using peripheral displays.
